# FASCICULE DE BREVET EUROPEEN

(11) **EP 2 135 638 B1**
(45) Date de publication et mention de la délivrance du brevet: **11.03.2015**
(21) Numéro de dépôt: 09162310.8
(22) Date de dépôt: 09.06.2009
(51) Int. Cl.: A61M 25/00, A61M 25/09, A61N 1/05

(54) **Mandrin préformé de guidage d'une sonde en contact avec la paroi du septum**
Vorgeformte Drehspindel zum Lenken einer Sonde in Kontakt mit der Septumswand
Preformed mandrel for guiding a probe in contact with the wall of the septum

(30) Priorité: 20.06.2008 FR 0803446
(43) Date de publication de la demande: 23.12.2009
(73) Titulaire: ELA MEDICAL, 92541 Montrouge (FR)
(72) Inventeur: Ollivier, Jean-François, 91190, VILLIERS LE BACLE (FR)
(74) Mandataire: Dupuis-Latour, Dominique

(56) Documents cités:
- EP-A- 0 778 044
- EP-A- 1 920 795
- WO-A-91/15152
- WO-A-94/20165
- US-A- 4 716 757

## Description

L'invention concerne la mise en place des sondes intracorporelles, notamment les sondes de détection/stimulation cardiaque des "dispositifs médicaux implantables actifs" tels que définis par la directive 90/385/CEE du 20 juin 1990 du Conseil des communautés européennes, plus précisément des implants cardiaques de stimulation, de resynchronisation, de cardioversion et/ou de défibrillation.

L'invention concerne plus précisément l'implantation d'un type particulier de sonde intracardiaque, destinée à être placée en appui contre la cloison interventriculaire ou interauriculaire, dite "septum cardiaque".

De telles sondes sont notamment utilisées avec les dispositifs multisite de stimulation ou de resynchronisation, pour stimuler les cavités gauches, auriculaire et/ou ventriculaire en fonction de la configuration et du placement de la sonde.

Il s'agit de sondes pourvues d'une vis d'ancrage, généralement une vis rétractable, permettant la fixation de la tête de sonde dans la paroi du septum, de sorte que l'électrode de la partie distale de la sonde soit appliquée de façon sensiblement perpendiculaire à cette paroi.

Compte tenu de cette configuration spécifique, le placement de ces sondes septales est une opération particulièrement délicate - à la différence des sondes de stimulation des cavités droites, qui sont simplement poussées jusqu'à atteindre le fond du ventricule.

Ces sondes septales sont, comme les autres sondes intracardiaques, introduites par le réseau veineux, soit via la veine céphalique droite et la veine cave supérieure ("abord droit"), soit via la veine céphalique gauche et la veine cave supérieure ("abord gauche").

Dans le cas d'une sonde conventionnelle de stimulation des cavités droites, la sonde est ensuite poussée jusqu'en fond de ventricule.

En revanche, pour une sonde septale, une fois atteint l'intérieur de la cavité, il est nécessaire d'orienter l'extrémité distale de la sonde perpendiculairement à la cloison du septum, puis appuyer cette extrémité contre la paroi au site de stimulation choisi pour y ancrer par vissage la tête de sonde portant l'électrode.

Pour guider aisément cette extrémité distale au moment de l'implantation vers le site de stimulation choisi, et dans une direction sensiblement perpendiculaire à celui-ci, la sonde doit être relativement rigide. Pour cela, le chirurgien introduit au préalable dans la gaine creuse, souple, du corps de sonde un mandrin ou "stylet" en forme de fil flexible. Le mandrin est muni, à son extrémité proximale émergeant de la sonde, d'une poignée de manoeuvre permettant au chirurgien d'imprimer des mouvements de rotation et translation du mandrin à l'intérieur de la gaine. La sonde, rigidifiée par le mandrin, peut alors être introduite dans le réseau veineux, puis son extrémité distale orientée par rotation de l'extrémité proximale du mandrin. De cette manière, le chirurgien fait tourner la partie d'extrémité de la tête de sonde et dirige cette dernière, qui présente une forme courbe, vers le site d'implantation du septum. Une fois le site atteint, le chirurgien ancre la tête de sonde par rotation de la vis d'ancrage qui vient pénétrer les tissus de la cloison septale.

A la différence des sondes de stimulation des cavités droites dont le positionnement est relativement aisé (placement en fond de ventricule), dans le cas d'une sonde dont la tête doit être ancrée sur la paroi septale, la diversité des voies d'accès veineuses et de la morphologie cardiaque rendent difficile l'accès au septum et le placement de la tête de sonde contre la paroi de ce septum.

Pour tenir compte de ces particularités, les chirurgiens tentent de définir leur propre conformation distale de mandrin, par déformation plastique de l'extrémité distale de celui-ci, de manière à donner à l'extrémité correspondante de la sonde, une fois introduite dans la cavité, une courbure facilitant l'approche et l'accostage de la paroi septale.

Le EP 1 920 795 A1 décrit un mandrin dont l'extrémité distale est formée d'un fil élastique préformé selon une configuration tridimensionnelle particulière, incluant une succession d'arcs curvilignes orientés dans des plans différents. Une fois le mandrin inséré dans la sonde et l'ensemble introduit jusque dans le ventricule et déployé dans celui-ci, cette configuration particulière permet de donner à la sonde une forme telle que celle-ci se tourne naturellement dans la direction recherchée. Le réglage fin de la position de la tête de sonde sera obtenu en poussant plus ou moins la poignée de manoeuvre située à l'extrémité opposée du mandrin : grâce à la succession des divers arcs curvilignes, le mouvement axial du mandrin dans la sonde se transformera en une rotation spontanée de celle-ci, sans qu'il soit besoin d'appliquer et de transmettre un couple de rotation. En effet, dans ce dispositif, la partie proximale est réalisée en un matériau déformable de façon permanente (de manière à donner une "mémoire" à la forme du mandrin), qui n'est pas destiné à transmettre le couple du fait de sa relative plasticité. Il est par ailleurs à noter que, dans ce dispositif, l'intégralité des conformations curvilignes du mandrin (et donc de la sonde) est localisée, et utilisée, dans le ventricule droit, c'est-à-dire dans le coeur, car il s'agit de permettre un réglage fin de l'orientation de la tête de sonde par une translation axiale du mandrin.

Le EP 0 778 044 A2 décrit un accessoire de type "guidewire" ou fil-guide, adapté notamment à une technique dite *Over-The-Wire* ou OTW, où le fil-guide traverse de part en part la gaine de sonde sur toute sa longueur, en débouchant et en émergeant côté distal. Pour rendre son extrémité atraumatique, et permettre sa progression directement dans une veine sans risque, le fil-guide est pourvu à son extrémité distale d'une boule ou d'une spire. En rentrant plus ou moins le fil-guide dans la gaine de sonde, il est possible de raidir cette dernière et de lui donner une courbure particulière, modifiable, permettant de l'orienter plus aisément dans la cavité cardiaque ou dans le réseau coronaire (mais donc toujours dans sa partie située dans le coeur). Une application typique d'un tel fil-guide concerne le placement d'une sonde dans le sinus coronaire, par une technique consistant à faire pénétrer le mandrin dans le sinus coronaire puis dans le réseau veineux coronaire, puis en faisant progressivement coulisser la sonde sur ce guide jusqu'à atteindre la position finale recherchée.

On a également proposé des dispositifs plus complexes, avec deux mandrins emmanchés l'un dans l'autre, permettant de modifier l'angle d'ouverture de la partie courbée de l'extrémité distale. Cette solution est relativement onéreuse et complexe, et souvent n'est pas adaptée à la fonction recherchée, dans la mesure où il n'est possible de modifier que l'angle d'ouverture de la partie d'extrémité courbée, et non le rayon de courbure lui-même.

En outre, quel que soit le dispositif utilisé, un phénomène typique, bien connu des praticiens, se produit lors de la rotation du mandrin à l'intérieur de la gaine creuse de la sonde : une rotation progressive, à l'extrémité proximale, de la poignée du mandrin se traduit, à l'extrémité distale opposée, tout d'abord par une rotation très faible puis, passé un certain seuil, par un saut brutal, et ainsi de suite au fur et à mesure de la rotation de la poignée du mandrin. Ce phénomène mécanique de relaxation résulte d'une accumulation progressive de contraintes mécaniques de torsion du mandrin dans la gaine de sonde, puis de libération brusque de ces contraintes avec saut de l'extrémité distale, ressentie par le praticien sous forme d'un "cliquetis" lors d'une rotation du mandrin. Il en résulte une incapacité à contrôler finement le mouvement de la partie distale du mandrin, et donc de diriger correctement la sonde vers le septum avec toute la progressivité et la précision nécessaires.

Ce phénomène de cliquetis a pour origine la position d'équilibre mécanique en torsion du mandrin dans la lumière de la gaine creuse de la sonde, cette dernière épousant la forme du réseau veineux dans lequel elle a été introduite. Dans la mesure où l'insertion de la sonde, rigidifiée par le mandrin, engendre souvent une déformation permanente, bien que faible, du corps du mandrin, cette déformation permanente tente systématiquement de s'inscrire dans la trajectoire imposée par la morphologie veineuse (qui elle-même dépend de la voie d'accès, droite ou gauche), du fait du principe mécanique de base de moindre énergie en torsion.

L'un des buts de l'invention est de proposer une solution permettant de pallier ce phénomène, en proposant une nouvelle technique assurant un auto-positionnement basé sur l'anatomie des veines d'accès et partant, une transmission efficace de la torsion imprimée par la poignée de manoeuvre, jusqu'à l'extrémité distale de la sonde, pour un placement aisé de la tête de sonde contre la paroi du septum, en direction du site de stimulation choisi par le chirurgien.

On notera que l'invention, si elle est définie ici pour le placement d'une sonde, s'applique aussi bien à d'autres éléments analogues, notamment au placement d'un cathéter transseptal pour réaliser par exemple un accès à une cavité gauche.

La solution de l'invention consiste à mettre en oeuvre un mandrin de guidage du type général connu, divulgué par le EP 1 920 795 A1 précité, correspondant au préambule de la revendication 1.

L'invention est définie par les caractéristiques énoncées dans la partie caractérisante de la revendication 1. Les sous-revendications visent des formes de réalisation subsidiaires avantageuses.

En d'autres termes, au lieu de lutter contre le phénomène d'accumulation des contraintes et de libération brusque résultant du principe mécanique de moindre énergie en torsion, l'invention propose d'utiliser ce phénomène mécanique, simple et prédictible, pour assurer un positionnement de la tête de sonde dans la direction du septum.

En effet, la conformation particulière donnée, selon l'invention, au mandrin lui permet de coïncider mécaniquement avec la courbure du réseau veineux, notamment de la veine cave supérieure et de la veine céphalique (droite ou gauche). Cette courbure, inscrite naturellement dans la courbure anatomique, permet d'orienter automatiquement la partie distale du mandrin, et donc la tête de sonde, dans la direction souhaitée. L'orientation angulaire fine sera ajustée par rotation de la poignée du mandrin, avec une plage de manoeuvre, de l'ordre de ± 20° à ± 40°, réalisée de manière progressive autour de la position d'équilibre, avant que ne se produise le saut du mandrin.

La solution proposée par l'invention est particulièrement simple et économique à mettre en oeuvre, à la différence des systèmes complexes à double mandrin qui ont pu être proposés par la technique antérieure.

En outre, le mandrin de l'invention peut être préformé directement en usine, en ne nécessitant qu'un ajustement fin par le chirurgien (déformation plastique de l'extrémité distale). En d'autres termes, la conformation générale est réalisée par le fabricant et affinée par le chirurgien au moment de l'implantation. L'ajustement final portera essentiellement sur l'extrémité distale, tandis que les autres courbures (rayon, ouverture angulaire, position sur la longueur du mandrin, position angulaire des plans des courbures, etc.) sont conformées en usine.

Dans une variante de mise en oeuvre, le mandrin n'est pas préformé, mais il est mis à disposition du chirurgien des outils de conformation simples utilisables au bloc opératoire, par exemple des gabarits présentant différents rayons de courbure thermoformés sur le "blister" dans lequel est conditionné le mandrin, avec des repères angulaires appropriés.

L'invention a également pour objet un nécessaire comportant un mandrin de guidage pour l'introduction dans le réseau veineux, puis l'orientation et le guidage de l'extrémité distale d'une sonde jusqu'au contact de la paroi du septum auriculaire ou ventriculaire.

Le nécessaire peut comprendre le fil déjà préformé suivant une configuration telle qu'exposée ci-dessus. En variante, il peut comprendre un fil non préformé, essentiellement rectiligne à l'état non sollicité, et un gabarit de formage dudit fil suivant une configuration telle qu'exposée ci-dessus, le gabarit pouvant être en particulier un relief formé sur un conditionnement du mandrin.

On va maintenant décrire un exemple de réalisation de l'invention, en référence aux dessins annexés où les mêmes références numériques désignent d'une figure à l'autre des éléments identiques ou fonctionnellement semblables.

La Figure 1 illustre schématiquement les voies d'accès et le mode d'implantation d'une sonde placée contre le septum cardiaque.

Les Figures 2 et 3 illustrent la conformation particulière du mandrin selon l'invention, pour une implantation adaptée respectivement à un abord droit et à un abord gauche.

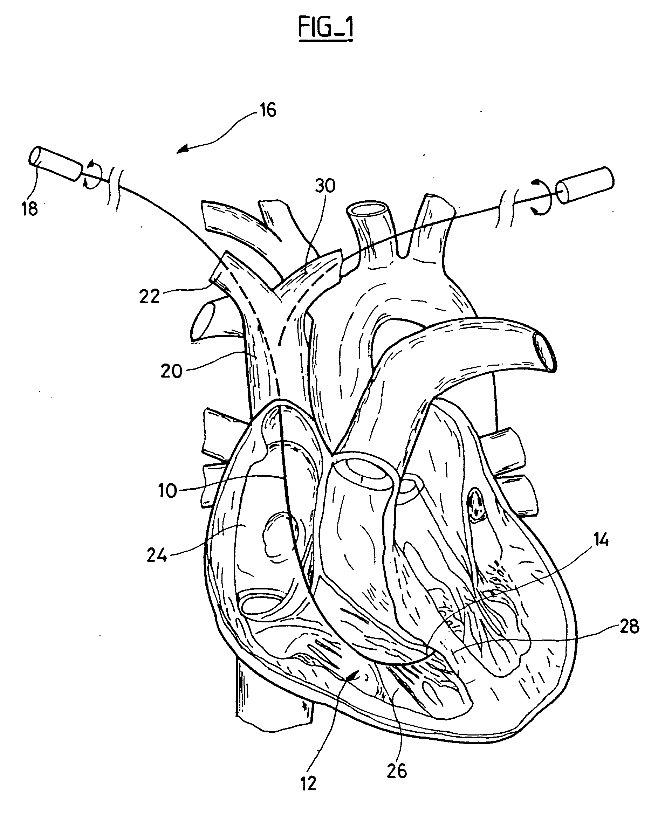

Sur les Figures 1 à 3, la référence 10 désigne de façon générale un mandrin enfilé dans la lumière centrale de la gaine creuse d'une sonde intracardiaque de manière que cette dernière, dont la structure est très souple, épouse la forme donnée par le mandrin introduit à l'intérieur.

La partie distale 12 débouche dans le ventricule droit, et l'extrémité distale 14 correspond à la tête de sonde, qui est destinée à être implantée contre la paroi ou septum interventriculaire.

A l'extrémité opposée, proximale 16, le mandrin 10 est pourvu d'une poignée de manoeuvre 18 qui permet notamment le contrôle, par transmission de l'effort de torsion, de la partie distale 12 pour orienter celle-ci, et donc la sonde dans laquelle est enfilé le mandrin, en direction du site de stimulation choisi.

Le mandrin est constitué d'un fil flexible en matériau relativement rigide mais élastiquement déformable, tel qu'un fil d'acier inoxydable AISI 302 ou AISI 304 de diamètre typiquement compris entre 0,30 mm et 0,45 mm. Le fil est élastiquement déformable en flexion, et présente en torsion une rigidité suffisante pour permettre la transmission sur toute la longueur du fil d'un mouvement de rotation imprimé par le chirurgien au moyen de la poignée 18.

La sonde pourvue de son mandrin est introduite dans la cavité cardiaque via la veine cave supérieure 20 et la veine céphalique droite 22, pour atteindre l'oreillette droite 24 puis le ventricule 26 (dans le cas d'une implantation contre le septum interventriculaire). Par pivotement de la poignée 18, le praticien oriente alors la tête de sonde en direction du septum interventriculaire 28 jusqu'à accoster la paroi de ce septum, où il pourra ensuite ancrer la tête de sonde par vissage de celle-ci dans le tissu du septum.

Une autre approche est possible, via cette fois la veine céphalique gauche 30 et la veine cave supérieure 20. Ce dernier cas est dénommé "abord gauche", tandis que le précédent est dénommé "abord droit".

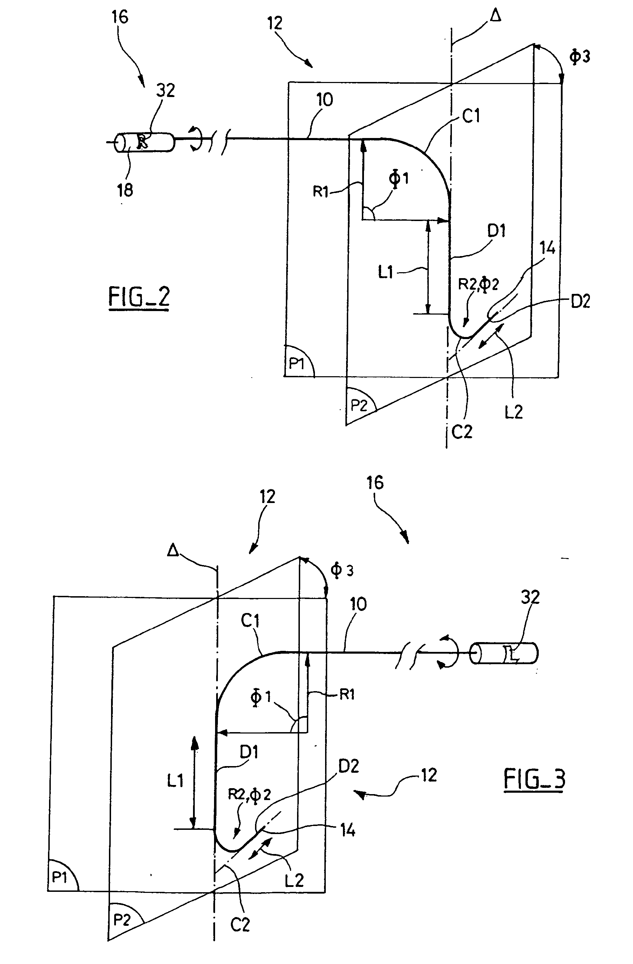

On a illustré sur les Figures 2 et 3 la conformation particulière donnée à la partie distale 12 du mandrin 10 selon l'invention. Cette configuration est différente selon l'abord choisi, la Figure 2 correspondant à un abord droit et la Figure 3 à un abord gauche.

Cette conformation spécifique est de préférence donnée au mandrin en usine, le mandrin étant alors livré préformé, de manière à présenter la courbure que l'on va décrire lorsqu'il est déployé à l'état libre, en l'absence de toute sollicitation (c'est-à-dire notamment avant introduction dans la lumière centrale de la gaine creuse de la sonde).

La partie distale 12 comporte successivement, depuis la partie centrale jusqu'à l'extrémité distale 14 :
- une première partie curviligne C1, de rayon de courbure R1 et d'angle d'ouverture Φ1 ;
- une première partie rectiligne D1, de longueur L1 ;
- une deuxième partie curviligne C2, de rayon de courbure R2 et d'angle d'ouverture Φ2 ; et
- une seconde partie rectiligne D2, de longueur L2.

On notera que, dans l'exemple illustré, les parties curvilignes sont des parties en arc de cercle, c'est-à-dire à rayon de courbure constant, mais que d'autres formes curvilignes peuvent être envisagées aussi bien, notamment des parties curvilignes conformées suivant un arc d'ellipse. Dans le cadre de la présente invention, le terme "curviligne" doit donc être entendu dans son acception la plus générale.

De préférence, les parties curvilignes C1 et C2 ne sont pas coplanaires, mais au contraire disposées dans deux plans respectifs P1, P2 formant entre eux un angle Φ3 définissant un dièdre dont l'axe Δ comprend la première partie rectiligne D1.

Dans le cas d'un mandrin destiné à un abord droit (Figure 2), les deux parties curvilignes C1 et C2 sont disposées en éloignement mutuel, c'est-à-dire de part et d'autre d'un demi-plan contenant la partie rectiligne D1.

Dans le cas d'un mandrin destiné à un abord gauche (Figure 3), les deux parties curvilignes C1 et C2 sont disposées en rapprochement mutuel, c'est-à-dire du même côté d'un demi-plan contenant la partie rectiligne D1.

Si les mandrins sont préformés en usine, le conditionnement peut contenir l'un et l'autre types de mandrin, et le chirurgien choisira celui qui est adapté à la voie d'abord, droite ou gauche, qu'il envisage. Dans ce cas, il est avantageux de prévoir sur la poignée un marquage 32, par exemple une inscription "L" ou "R", ou bien un code de couleur permettant de distinguer plus facilement l'un et l'autre mandrin.

Les sept paramètres définissant la forme particulière du mandrin selon l'invention peuvent varier dans les plages suivantes :
Pour la première partie curviligne :
   R1 ≤ 200 mm, de préférence R1 = 40 à 60 mm
   Φ1 ≥ 10°, de préférence Φ1 = 70 à 110°
Pour la première partie rectiligne :
   L1 ≥ 10 mm, de préférence L1 = 30 à 70 mm
Pour la seconde partie curviligne :
   R2 = 10 à 15 mm
   Φ2 = 20 à 40°
Pour la seconde partie rectiligne :
   L2 = 0 à 15 mm (ce qui signifie que cette deuxième partie rectiligne peut être omise, l'extrémité distale 14 se situant immédiatement à l'extrémité libre de la deuxième partie curviligne C2)
Angle mutuel des plans P1 et P2 :
   Φ3 = 0 à 45°, de préférence Φ3 = 10 à 30°.
Si l'on prend les valeurs extrêmes de rayon de courbure et d'angle, on peut calculer la longueur développée de l'arc curviligne correspondant :
   - pour la première partie curviligne C1 : 49 à 115 mm,
   - pour la seconde partie curviligne C2 : 3 à 10 mm.

Soit, avec une longueur de première partie rectiligne D1 comprise entre 30 et 70 mm, et une longueur de seconde partie rectiligne (facultative) comprise entre 0 et 15 mm, un développement total compris entre :
- 82 et 195 mm en l'absence de seconde partie rectiligne, et
- 82 à 210 mm en présence d'une seconde partie rectiligne.

Ces valeurs extrêmes de longueur développée reflètent le fait que la succession de parties curvilignes et rectilignes doit couvrir la courbure anatomique du réseau veineux dans lequel sera introduite la sonde pourvue de son mandrin, et non la seule cavité cardiaque (où ne s'étendent que les secondes parties rectilignes et curvilignes).

## Revendications

1. Un mandrin de guidage, destiné à rigidifier une sonde pour permettre l'introduction dans le réseau veineux, comportant :
- un fil flexible (10), destiné à être introduit par son extrémité distale dans une lumière centrale de ladite sonde, et
- une poignée de manoeuvre (18), solidaire du fil à l'extrémité proximale (16) de ce fil,
le fil étant élastiquement déformable en flexion et présentant en torsion une rigidité suffisante pour permettre la transmission sur toute la longueur du fil d'un mouvement de rotation imprimé par la poignée,
le fil présentant à l'état non sollicité, dans sa partie distale (12), successivement : une première partie curviligne (C1), une première partie rectiligne (D1), une seconde partie curviligne (C2) et une seconde partie rectiligne (D2),
la première partie curviligne (C1) et la seconde partie curviligne (C2) s'étendant dans des plans respectifs (P1, P2) faisant entre eux un angle (Φ3) définissant un dièdre dont l'axe (Δ) comprend la première partie rectiligne (D1),
mandrin **caractérisé en ce que** :
- la première partie curviligne (C1) présente un rayon de courbure (R1) inférieur ou égal à 200 mm et un angle d'ouverture (Φ1) d'au moins 10° ;
- la première partie rectiligne (D1) présente une longueur (L1) d'au moins 10 mm;
- la seconde partie curviligne (C2) présente un rayon de courbure (R2) compris entre 10 et 15 mm et un angle d'ouverture (Φ2) compris entre 20° et 40° ;
- la seconde partie rectiligne (D2) présente une longueur (L2) inférieure ou égale à 15 mm ;
- l'angle (Φ3) du dièdre est inférieur à 45° ; et
- la longueur totale de la première partie curviligne, de la première partie rectiligne et de la seconde partie curviligne est comprise entre 82 et 195 mm,
de manière à coïncider avec la courbure anatomique du réseau veineux dans lequel sera introduite la sonde pourvue de son mandrin,
et permettre ainsi l'orientation et le guidage de l'extrémité distale de la sonde jusqu'au contact de la paroi du septum (28) ventriculaire.

2. Le mandrin de la revendication 1, dans lequel la première partie curviligne (C1) et la seconde partie curviligne (C2) sont configurées l'une par rapport à l'autre, et par rapport à la première partie rectiligne (D1), de manière à s'étendre :
- en éloignement mutuel, de part et d'autre d'un demi-plan contenant la première partie rectiligne (D1), pour un mandrin destiné à l'introduction dans le réseau veineux via la veine céphalique droite (22), ou bien
- en rapprochement mutuel, d'un même côté d'un demi-plan contenant la première partie rectiligne (D1), pour un mandrin destiné à l'introduction dans le réseau veineux via la veine céphalique gauche (30).

3. Le mandrin de la revendication 2, dans lequel la poignée de manoeuvre (18) porte un marquage (32) indiquant si le mandrin est un mandrin destiné à l'introduction dans le réseau veineux via la veine céphalique droite (22) ou la veine céphalique gauche (30).

4. Le mandrin de la revendication 1, dans lequel l'angle (Φ3) du dièdre est compris entre 10 et 30°.

5. Le mandrin de la revendication 1, dans lequel la première partie curviligne (C1) présente un rayon de courbure (R1) compris entre 40 et 60 mm.

6. Le mandrin de la revendication 1, dans lequel la première partie curviligne (C1) présente un angle d'ouverture (Φ1) compris entre 70° et 110°.

7. Le mandrin de la revendication 1, dans lequel la première partie rectiligne (D1) présente une longueur comprise entre 30 et 70 mm.

8. Un nécessaire pour l'introduction dans le réseau veineux, puis l'orientation et le guidage de l'extrémité distale d'une sonde jusqu'au contact de la paroi du septum ventriculaire,
ce nécessaire comportant :
- un mandrin de guidage comprenant un fil préformé suivant une configuration selon l'une des revendications 1 à 7.

9. Un nécessaire pour l'introduction dans le réseau veineux, puis l'orientation et le guidage de l'extrémité distale d'une sonde jusqu'au contact de la paroi du septum ventriculaire,
ce nécessaire comportant :
- un mandrin de guidage comprenant un fil non préformé, essentiellement rectiligne à l'état non sollicité, et
- un gabarit de formage dudit fil suivant une configuration selon l'une des revendications 1 à 7.

10. Le nécessaire de la revendication 9, dans lequel ledit gabarit est un relief formé sur un conditionnement du mandrin.

## Patentansprüche

1. Führungsspindel, die dazu bestimmt ist, eine Sonde zu versteifen, um die Einführung in das Venennetz zu ermöglichen, umfassend:
- einen flexiblen Draht (10), der dazu bestimmt ist, mit seinem distalen Ende in ein mittiges Lumen der Sonde eingeführt zu werden, und
- einen Bediengriff (18), der mit dem Draht am proximalen Ende (16) dieses Drahts fest verbunden ist,
wobei der Draht elastisch biegeverformbar ist und eine ausreichende Torsionssteifigkeit aufweist, um die Übertragung einer Drehbewegung, die vom Griff ausgeht, auf die gesamte Länge des Drahts zu ermöglichen;
wobei der Draht im unbelasteten Zustand in seinem distalen Abschnitt (12), nacheinander Folgendes aufweist: einen ersten krummlinigen Abschnitt (C1), einen ersten geradlinigen Abschnitt (D1) und einen zweiten krummlinigen Abschnitt (C2) und einen zweiten geradlinigen Abschnitt (D2),
wobei sich der erste krummlinige Abschnitt (C1) und der zweite krummlinige Abschnitt (C2) in jeweiligen Ebenen (P1, P2) erstrecken, die einen Winkel (Φ3) zueinander einnehmen, der ein Dieder definiert, dessen Achse (Δ) den ersten geradlinigen Abschnitt (D1) umfasst,
wobei die Spindel **dadurch gekennzeichnet ist, dass**:
- der erste krummlinige Abschnitt (C1) einen Krümmungsradius (R1) kleiner oder gleich 200 mm und einen Öffnungswinkel (Φ1) von mindestens 10° aufweist;
- der erste geradlinige Abschnitt (D1) eine Länge (L1) von mindestens 10 mm aufweist;
- der zweite krummlinige Abschnitt (C2) einen Krümmungsradius (R2) im Bereich zwischen 10 und 15 mm und einen Öffnungswinkel (Φ2) im Bereich zwischen 20 und 40 aufweist;
- der zweite geradlinige Abschnitt (D2) eine Länge (L2) kleiner oder gleich 15 mm aufweist;
- der Winkel (Φ3) des Dieders kleiner als 45° ist, und
- die Gesamtlänge des ersten krummlinigen Abschnitts, des ersten geradlinigen Abschnitts und des zweiten krummlinigen Abschnitts im Bereich zwischen 82 und 195 mm liegt,
um mit der anatomischen Krümmung des Venensystems übereinzustimmen, in das die Sonde, die mit ihrer Spindel versehen ist, eingeführt wird,
und um so die Ausrichtung und die Führung des distalen Endes der Sonde bis zum Kontakt mit der ventrikularen Septumswand (28) zu ermöglichen.

2. Spindel nach Anspruch 1, wobei der erste krummlinige Abschnitt (C1) und der zweite krummlinige Abschnitt (C2) zueinander und bezogen auf den ersten geradlinigen Abschnitt (D1) ausgebildet sind, um sich:
- in gegenseitiger Entfernung beiderseits einer Halbebene, die den ersten geradlinigen Abschnitt (D1) enthält, für eine Spindel, die für die Einführung in das Venennetz system über die rechte Kopfvene (22) bestimmt ist, oder auch
- in gegenseitiger Annäherung auf der gleichen Seite einer Halbebene, die den ersten geradlinigen Abschnitt (D1) enthält, für eine Spindel, die für die Einführung in das Venennetz über die linke Kopfvene (30) bestimmt ist, zu erstrecken.

3. Spindel nach Anspruch 2, wobei der Bediengriff (18) eine Markierung (32) trägt, die angibt, ob die Spindel eine Spindel ist, die für die Einführung in das Venennetz über die rechte Kopfvene (22) oder über die linke Kopfvene (30) bestimmt ist.

4. Spindel nach Anspruch 1, wobei der Winkel (Φ3) des Dieders im Bereich zwischen 10 und 30° liegt.

5. Spindel nach Anspruch 1, wobei der erste krummlinige Abschnitt (C1) einen Krümmungsradius (R1) im Bereich zwischen 40 und 60 mm aufweist.

6. Spindel nach Anspruch 1, wobei der erste krummlinige Abschnitt (C1) einen Öffnungswinkel (Φ1) im Bereich zwischen 70 und 110° aufweist.

7. Spindel nach Anspruch 1, wobei der erste geradlinige Abschnitt (D1) eine Länge im Bereich zwischen 30 und 70 mm aufweist.

8. Kit für die Einführung in das Venennetz, dann die Ausrichtung und die Führung des distalen Endes einer Sonde bis zum Kontakt mit der ventrikularen Septumswand,
wobei dieser Kit Folgendes umfasst:
- eine Führungsspindel, die einen vorgeformten Draht gemäß einer Konfiguration nach einem der Ansprüche 1 bis 7 umfasst.

9. Kit für die Einführung in das Venennetz, dann die Ausrichtung und die Führung des distalen Endes einer Sonde bis zum Kontakt mit der ventrikularen Septumswand,
wobei dieser Kit Folgendes umfasst:
- eine Führungsspindel, die einen nicht vorgeformten Draht umfasst, der im unbelasteten Zustand im Wesentlichen geradlinig ist, und
- eine Formschablone für den Draht gemäß einer Konfiguration nach einem der Ansprüche 1 bis 7.

10. Kit nach Anspruch 9, wobei die Schablone ein Relief ist, das auf einer Verpackung der Spindel gebildet wird.

## Claims

1. A guiding mandrel, intended to stiffen a lead for allowing the introduction into the venous system, including:
- a flexible wire (10), intended to be introduced by its distal end into a central lumen of said lead, and
- an operating handle (18), integral with the wire at the proximal end (16) of this wire,
the wire being elastically deformable in flexion and having in torsion a sufficient stiffness to allow the transmission, over the whole length of the wire, of a rotational movement imparted by the handle,
the wire having, at the non-stressed state, in its distal part (12), successively: a first curvilinear part (C1), a first rectilinear part (D1), a second curvilinear part (C2) and a second rectilinear part (D2),
the first curvilinear part (C1) and the second curvilinear part (C2) extending in respective planes (P1, P2) forming between each other a angle (Φ3) defining a dihedron whose axis (Δ) comprises the first rectilinear part (D1),
the mandrel being **characterized in that**:
- the first curvilinear part (C1) has a radius of curvature (R1) lower than or equal to 200 mm and an angle of opening (Φ1) of at least 10°;
- the first rectilinear part (D1) has a length (L1) of at least 10 mm;
- the second curvilinear part (C2) has a radius of curvature (R2) comprised between 10 and 15 mm and an angle of opening (Φ1) comprised between 20° and 40°;
- the second rectilinear part (D2) has a length (L2) lower than or equal to 15 mm;
- the angle (Φ3) of the dihedron is lower than 45°; and
- the total length of the first curvilinear part, of the first rectilinear part and of the second curvilinear part is comprised between 82 and 195 mm,
so as to match the anatomic curvature of the venous system into which the lead provided with its mandrel will be introduced,
and to hence allow the orientation and the guiding of the distal end of the lead up to contact with the wall of the ventricular septum (28).

2. The mandrel of claim 1, wherein the first curvilinear part (C1) and the second curvilinear part (C2) are configured with respect to each other, and with respect to the first rectilinear part (D1), so as to move:
- away from each other, on either side of a half-plane containing the first rectilinear part (D1), for a mandrel intended for the introduction into the venous system via the right cephalic vein (22), or
- closer to each other, on a same side of a half-plane containing the first rectilinear part (D1), for a mandrel intended for the introduction into the venous system via the left cephalic vein (30).

3. The mandrel of claim 2, wherein the operating handle (18) carries a mark (32) indicating if the mandrel is a mandrel intended for the introduction into the venous system via the right cephalic vein (22) or the left cephalic vein (30).

4. The mandrel of claim 1, wherein the angle (Φ3) of the dihedron is comprised between 10 and 30°.

5. The mandrel of claim 1, wherein the first curvilinear part (C1) has a radius of curvature (R1) comprised between 40 and 60 mm.

6. The mandrel of claim 1, wherein the first curvilinear part (C1) has an angle of opening (Φ1) comprised between 70° and 110°.

7. The mandrel of claim 1, wherein the first rectilinear part (D1) has a length comprised between 30 and 70 mm.

8. A kit for the introduction into the venous system, then the orientation and the guiding of the distal end of a lead up to contact with the wall of the ventricular septum,
this kit including:
- a guiding mandrel comprising a wire pre-shaped in a configuration according one of claims 1 to 7.

9. A kit for the introduction into the venous system, then the orientation and the guiding of the distal end of a lead up to contact with the wall of the ventricular septum,
this kit including:
- a guiding mandrel comprising a non-pre-shaped wire, essentially rectilinear at the non-stressed state, and
- a template for shaping said wire in a configuration according to one claims 1 to 7.

10. The kit of claim 9, wherein said template is a relief formed on a packaging of the mandrel.
